# EUROPÄISCHE PATENTSCHRIFT

(11) **EP 2 720 607 B1**
(45) Veröffentlichungstag und Bekanntmachung des Hinweises auf die Patenterteilung: **18.11.2015**
(21) Anmeldenummer: 12729937.8
(22) Anmeldetag: 14.06.2012
(51) Int. Cl.: A61B 5/01, F24F 11/00, A61B 5/00

(54) **VERFAHREN UND VORRICHTUNG ZUR ERFASSUNG DER THERMISCHEN BEHAGLICHKEIT**
METHOD AND DEVICE FOR DETECTING THERMAL COMFORT
PROCÉDÉ ET DISPOSITIF DE DÉTECTION DU CONFORT THERMIQUE

(30) Priorität: 15.06.2011 DE 102011077522
(43) Veröffentlichungstag der Anmeldung: 23.04.2014
(73) Patentinhaber: Fraunhofer Gesellschaft zur Förderung der angewandten Forschung E.V., 80686 München (DE); Universität Stuttgart, 70174 Stuttgart (DE)
(72) Erfinder: VAN TREECK, Christoph, 87754 Kammlach (DE); SEDLBAUER, Klaus, 70192 Stuttgart (DE); WÖLKI, Daniel, 83624 Otterfing (DE)
(74) Vertreter: Andrae | Westendorp Patentanwälte Partnerschaft
(86) Internationale Anmeldenummer: PCT/EP2012/061375
(87) Internationale Veröffentlichungsnummer: WO 2012/172026

(56) Entgegenhaltungen:
- DE-U1-202008 010 875
- JP-A- 7 049 141
- ALAHMER A ET AL: "Vehicular thermal comfort models; a comprehensive review", APPLIED THERMAL ENGINEERING, PERGAMON, OXFORD, GB, Bd. 31, Nr. 6, 1. Dezember 2010 (2010-12-01), Seiten 995-1002, XP028143857, ISSN: 1359-4311, DOI: 10.1016/J.APPLTHERMALENG.2010.12.004 [gefunden am 2010-12-08]
- DE OLIVEIRA F ET AL: "Infrared Imaging Analysis for Thermal Comfort Assessment", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22. August 2007 (2007-08-22), Seiten 3373-3376, XP031336933, ISBN: 978-1-4244-0787-3
- QUAN JIN ET AL: "Predictive model of local and overall thermal sensations for non-uniform environments", BUILDING AND ENVIRONMENT, PERGAMON PRESS, OXFORD, GB, Bd. 51, 3. Dezember 2011 (2011-12-03), Seiten 330-344, XP028443970, ISSN: 0360-1323, DOI: 10.1016/J.BUILDENV.2011.12.005 [gefunden am 2011-12-13]

## Beschreibung

Die Erfindung betrifft eine Vorrichtung zur Erfassung der thermischen Behaglichkeit, welche zumindest eine die thermische Behaglichkeit repräsentierende Messgröße erzeugt. Weiterhin betrifft die Erfindung ein Verfahren, bei welchem zumindest eine die thermische Behaglichkeit repräsentierende Messgröße erzeugt wird. Verfahren und Vorrichtungen der eingangs genannten Art können zur bedarfsorientierten Steuerung oder Regelung von Lüftungs-, Heizungs- oder Klimaanlagen in Gebäuden, Flug- oder Fahrzeugen eingesetzt werden oder zur Visualisierung der thermischen Behaglichkeit.

Aus der JP 7049141 A ist eine gattungsgemäße Vorrichtung bekannt. Diese berücksichtigt neben der Lufttemperatur im zu klimatisierenden Raum auch die Luftfeuchte, die Geschwindigkeit einer Luftströmung und die von den Begrenzungsflächen des Raumes ausgehende Infrarotstrahlung zur Bestimmung der dem Raum zuzuführenden Luftmenge und/oder deren Temperatur. Darüber hinaus wird die Temperatur der Haut- und der Bekleidungsoberfläche zumindest eines Bewohners des zu klimatisierenden Raumes berührungslos gemessen, um hieraus den Wärmeeintrag durch die Person und den Isolationswert der Bekleidung zu ermitteln. Hierdurch kann das Raumklima noch besser auf den tatsächlichen Bedarf der Bewohner abgestimmt werden.

Dieses bekannte Verfahren weist jedoch den Nachteil auf, dass das Thermographiebild des Bewohners zunächst keine Rückschlüsse auf die thermische Behaglichkeit zulässt. So kann beispielsweise die Raumtemperatur abgesenkt werden, wenn der Isolationswert der vom Bewohner getragenen Kleidung sehr groß ist, unklar bleibt jedoch, ob diese kühlere Temperatur vom Bewohner toleriert oder als angenehm empfunden wird.

Der Erfindung liegt somit die Aufgabe zugrunde, die thermische Behaglichkeit automatisiert zu erfassen. Weiterhin liegt einer Ausführungsform der Erfindung die Aufgabe zugrunde, den Gesundheitszustand einer Person berührungslos zu erfassen.

Die Aufgabe wird erfindungsgemäß durch eine Vorrichtung gemäß Anspruch 1 und ein Verfahren gemäß Anspruch 10 gelöst.

Die Erfindung beruht auf dem Grundprinzip, die Oberflächentemperatur bzw. Infrarotsignatur von Personen mittels eines bildgebenden thermographischen Verfahrens digital zu erfassen, diese Daten durch optionale Informationen weiterer Sensorik zu ergänzen und mit einem Verfahren zur 3D Gestik- oder Bewegungserfassung zu kombinieren. Damit kann die Oberflächentemperaturinformation den entsprechenden Oberflächen eines segmentierten, dreidimensionale physiologischen Modells eines Menschen zugeordnet werden. Die segmentweise bekannten Temperaturinformationen können dann zur Bestimmung des Temperaturempfindens bzw. des Behaglichkeitsempfindens verwendet werden. Hierfür kann ein empirisches Behaglichkeitsmodell dienen, welches optional um ein Modell zur Erfassung instationärer Vorgänge erweitert werden kann.

Erfindungsgemäß wird vorgeschlagen, zur Erfassung der thermischen Behaglichkeit eine Bilderfassungseinrichtung mit einem vorgebbaren Erfassungsbereich einzusetzen. Die Bilderfassungseinrichtung erfasst die Bewegung zumindest einer Person, welche sich innerhalb des Erfassungsbereiches aufhält. Die Bilderfassungseinrichtung kann in einigen Ausführungsformen der Erfindung zumindest eine digitale Kamera enthalten, welche ein zweidimensionales Bild des Erfassungsbereiches auf einen Halbleitersensor abbildet. Der Halbleitersensor kann das zweidimensionale Bild in ein elektrisches Signal wandeln, welches als analoges oder digitales Signal weiterverarbeitet werden kann. Die Bilderfassungseinrichtung kann im sichtbaren Spektralbereich und/oder im infraroten Spektralbereich arbeiten. In einigen Ausführungsformen der Erfindung kann die Bilderfassungseinrichtung eine Mehrzahl von Kameras bzw. Kamerasystemen umfassen, um auf diese Weise eine drei-dimensionale Bewegungserfassung im Erfassungsbereich zu ermöglichen.

Der von der Bilderfassungseinrichtung erfasste Raum kann in einigen Ausführungsformen der Erfindung ein Innenraum eines .Gebäudes, eines Fahrzeuges oder eines Flugzeuges sein. In anderen Ausführungsformen der Erfindung kann der Erfassungsbereich eine Teilfläche bzw. ein Teilvolumen eines Innenraumes sein. In wiederum einer anderen Ausführungsform der Erfindung kann die Bilderfassungseinrichtung einen Teil eines Freigeländes erfassen, um die thermische Behaglichkeit der Personen auf dem Freigelände zu bestimmen.

Das Signal der Bilderfassungseinrichtung wird einer Bildauswerteeinrichtung zugeführt. Die Bildauswerteeinrichtung ermittelt aus den von der Bilderfassungseinrichtung erfassten Bildern zumindest einer Person zumindest eine Position im Raum und/oder eine Gestik und/oder eine Mimik und/oder anthropometrische Daten der zumindest einen Person. Sofern die Bilderfassüngseinrichtung Bilder zumindest einer Person fortlaufend erfasst, kann die Bildauswerteeinrichtung auch den zeitlichen Verlauf einer Position im Raum und/oder den zeitlichen Verlauf der Gestik und/oder den zeitlichen Verlauf der Mimik der zumindest einen Person bestimmen. Sofern sich unterschiedliche Personen im Erfassungsbereich der Bilderfassungseinrichtung aufhalten, kann die Bildauswerteeinrichtung auch anthropometrische Daten, Gestik und/oder Positionen einer Mehrzahl von Personen bestimmen.

Für die Zwecke der vorliegenden Beschreibung bezeichnen anthropometrische Daten Körpermaße, d.h. (Längen und Längenverhältnisse) und keine spezifischen Körperteile. Daten, welche die Form und den Aufbau des "menschlichen Körpers" betreffen, werden als morphologische Daten bezeichnet und lassen z.B. Rückschlüsse auf das Geschlecht einer Person zu. Anthropometrische Daten wie z.B. Größe einer Person, sind Daten, mit denen z.B. die Körperoberfläche abgeschätzt werden kann. Damit kann ein mathematisches Berechnungsmodell parametriert und angepasst werden.

Durch die Ableitung morphologischer Daten wie z.B. die Form von Körpersegmenten wie Gesicht, Arme, Beine etc. einer Person, welche durch die Bilderfassungseinrichtung erfasst werden können, kann z.B. auf Alter und Geschlecht einer Person zurückgeschlossen werden. Hierfür stehen bekannte Formalismen zur Verfügung. Solche Daten können dann wiederum als Input-Parameter für die Anpassung eines mathematischen Berechnungsmodells dienen, z.B. eines thermophysiologischen Modells. Die Bewegungserfassung ist erfindungsgemäß zunächst unabhängig von der Anthropometrie und der Morphologie.

In der Bildauswerteeinrichtung kann die Oberflächentemperaturinformation der Bilderfassungseinrichtung den Oberflächen eines segmentierten, dreidimensionalen physiologischen Modells eines Menschen zugeordnet werden. Durch die Segmentierung ist bekannt, welche Körperteile erfasst werden, womit eine Differenzierung zwischen benachbarten bekleideten und unbekleideten Körperstellen vorgenommen werden kann Dadurch kann beurteilt werden, zu welchen prozentualen Anteilen der Körper segmentweise bekleidet ist. Durch die Differenzierung und den parallelen Einsatz eines thermophysiologischen Modells kann mit einem durch Simulation ermittelten Wärmestrom der Bekleidungsisolationswert segmentweise berechnet werden. In einigen Ausführungsformen der Erfindung können Oberflächentemperaturen anderer, nicht bildtechnisch erfasster Bereiche, oder die Körperkerntemperatur mittels eines parallel eingesetzten thermophysiologischen Simulationsmodells mit hoher Genauigkeit berechnet werden. Das physiologische Modell kann durch die mittels Gestikerkennung abgeleitete Information über Bewegung, Morphologie bzw. Anthropometrie kalibriert werden. Durch die physiologischen Daten können ferner Aussagen zum Gesundheitszustand oder physiologischen Wärmehaushalt abgeleitet werden. Der Einfluss von Solarstrahlung wird, ebenso wie andere thermodynamisch auf den Körper einwirkende Energiegrößen (thermische Vorgeschichte), implizit berücksichtigt, indem der Ist-Zustand des Körpers durch die Infrarotdaten, Sensordaten sowie die über das thermophysiologische Modell ermittelten Daten beschrieben wird.

Nachfolgend werden die von der Bildauswerteeinrichtung bereitgestellten Daten einer Korrelationseinrichtung zugeführt, welche aus der Position im Raum und/oder der Gestik und/oder der Mimik der zumindest einen Person zumindest eine die thermische Behaglichkeit repräsentierende Messgröße erzeugt. Die segmentweise bekannten Temperaturinformationen können zur Bestimmung des Temperaturempfindens bzw. des Behaglichkeitsempfindens verwendet werden. Hierfür kann ein empirisches Behaglichkeitsmodell dienen, beispielsweise ein Modell nach ISO 14505.

In einigen Ausführungsformen kann die Bildauswerteeinrichtung aus der Gesten- bzw. Bewegungserkennung, d.h. der zeitlich und/oder ortsäufgelösten Erfassung der Position einer Person und der relativen Positionen ihrer Körperteile zueinander Informationen über den Aktivitätsgrad einer Person gewinnen, womit die metabolische Rate, d.h. der Energieumsatz, einer Person abgeschätzt werden kann. Beispielsweise ist eine sitzende Person weniger aktiv als eine sich bewegende oder stehende Person. Jedoch hat auch das Lenken eines KFZ eine höhere körperliche Belastung zur Folge, verglichen mit einer Person in Ruheposition, wie z.B. dem Beifahrer. Damit ist der Energieumsatz des Fahrers höher als des Beifahrers. Der Aktivitätsgrad kann zur Erfassung der thermischen Behaglichkeit verwendet werden. Beispielsweise kann eine ruhende Person eine höhere Luftfeuchte und/oder eine höhere Temperatur als angenehm empfinden als eine körperlich aktive Person.

In einigen Ausführungsformen der Erfindung kann aus der Gestik bzw. der Bewegung oder der Körperhaltung auf das Wohlbefinden der Person geschlossen werden. Beispielsweise kann steigende Unruhe der Person auf thermischen Dyskomfort schließen lassen. In wiederum einer anderen Ausführungsform der Erfindung kann das Lüftungsverhalten der Person im Raum erfasst werden. So kann das Öffnen eines Fensters darauf hindeuten, dass der Frischluftbedarf erhöht ist oder die Temperatur im Raum abgesenkt werden sollte. In diesem Fall ist die aktuelle thermische Behaglichkeit somit gering. Die Mimik lässt Rückschlüsse auf den aktuellen Wachheitsgrad und den Aktivitätsgrad zu. Die Mimik verrät beispielsweise über den Augenaufschlag, ob eine Person ermüdet ist oder wach. Das Reiben der Augen kann auf einen Ermüdungszustand hinweisen, wohingegen aktives Gestikulieren bei Diskussionen auf eine allgemeine Erhöhung des Aktivitätsgrads schließen lässt. Ein Anzeichen für verhaltensbasierte thermische Unbehaglichkeit ist z.B. das Ablegen/ Anziehen bzw. das generelle Vorhandensein eines Bekleidungsstücks. Dieser Vorgang kann durch die Bilderfassungseinrichtung mit der oben beschriebenen Methodik detektiert werden, indem der Bekleidungsisolationswert über die Analyse/Simulation von Temperatur- und Wärmestrominformationen bestimmt wird. Ein in der Korrelationseinrichtung implementiertes Modell zur Erkennung von Verhaltensmustern im Zusammenhang mit thermischer Behaglichkeit kann dann ein entsprechendes Ausgangssignal liefern, welches dann als Trigger für den gesamten Behaglichkeitsregelungsmechanismus verwendet werden kann. Das hier skizzierte Grundprinzip der Gestikerkennung kann leicht um weitere Beispiele erweitert werden, welche dann in der Bildauswerteeinrichtung einer konkreten Ausführungsform der Erfindung umgesetzt werden können.

Die erfindungsgemäße Vorrichtung zur Erfassung der thermischen Behaglichkeit erfasst somit nicht zwingend oder zumindest nicht ausschließlich die Temperatur der Hautoberfläche einer Person, sondern das konkrete Verhalten und damit die Auswirkungen des aktuellen Raumklimas auf das Wohlbefinden der Person. Damit kann die thermische Behaglichkeit mit vergrößerter Genauigkeit bestimmt werden. Es ergibt sich ein vollständiges Abbild einer Person hinsichtlich Aktivität, Bewegung, Bekleidungsisolationswert, Morphologie, Anthropometrie und physiologischem Wärmehaushalt einschließlich Haut-/ Bekleidungsoberflächen- und Körperkerntemperaturen. Mit all diesen Informationen kann somit eine Informationsverarbeitung zur zielgerichteten regelungstechnischen Ansteuerung von Raumkonditionierungs- oder Klimatisierungssystemen erfolgen um eine behaglichkeitsbasierte, lokale Regelung und Ansteuerung von Klimatisierungseinrichtungen zu erreichen.

Die auf diese Weise erhaltene, die thermische Behaglichkeit repräsentierende Messgröße kann zur Visualisierung der Behaglichkeit verwendet werden, als Sollwert in einer Lüftungs-, Heizungs- oder Klimaanlage, als Eingangsgröße einer Recheneinheit, welche einen Sollwert einer Lüftungs-, Heizungs- oder Klimaanlage bestimmt oder als Istwert in einer Lüftungs-, Heizungs- oder Klimaanlage. Eine Lüftungs-, Heizungs- oder Klimaanlage im Sinne der vorliegenden Erfindung kann eine personalisierte Einrichtung für einen Benutzer umfassen, beispielsweise eine Sitzheizung oder eine Sitzbelüftung oder zumindest einen einer Person oder einer Gruppe von Personen zugeordneten Luftausströmer. In anderen Ausführungsformen der Erfindung kann eine Lüftungs-, Heizungs-oder Klimaanlage unspezifisch auf einen gesamten Raum, einen Raumabschnitt, ein gesamtes Gebäude oder einen Gebäudeabschnitt wirken. In diesem Fall kann die Lüftungs-, Heizungs-oder Klimaanlage ein Zuluftelement, ein Abluftelement, einen Heizkörper, eine Bauteilkühlung oder -beheizung oder ähnliche Einrichtungen enthalten.

Die die thermische Behaglichkeit repräsentierende Messgröße kann in einigen Ausführungsformen der Erfindung ein predicted mean vote (PMV) nach DIN EN ISO 7730 sein.

Die Vorrichtung enthält zumindest einen Infrarotsensor zur Erfassung der Temperatur der Oberfläche der Person an zumindest einer Stelle, wobei die Daten des Infrarotsensors der Korrelationseinrichtung zuführbar sind. Die Oberflächentemperatur des Körpers der Person bzw. deren lokale Hauttemperatur stellt sich durch den Kontakt des Körpers mit dem diesen umgebenden Mikroklima ein. Somit kann die Wirkung des Mikroklimas bestimmt werden, welches die thermische Behaglichkeit wesentlich beeinflusst. Dadurch wird die Genauigkeit der die thermische Behaglichkeit repräsentierenden Messgröße weiter erhöht.

In einigen Ausführungsformen der Erfindung kann der empirische Zusammenhang zwischen dem lokalen Temperatur- bzw. Behaglichkeitsempfinden und der gemessenen Hauttemperatur durch psychopsychische Probandenversuche ermittelt werden. Somit kann aus der gemessenen Hauttemperatur und dem Verhalten der Person die aktuelle thermische Behaglichkeit mit großer Genauigkeit bestimmt werden.

Der Infrarotsensor ist dazu eingerichtet, ein thermografisches Bild zu erzeugen, indem die Temperatur an einer Mehrzahl von Stellen der Hautoberfläche erfasst wird. Dies kann in einigen Ausführungsformen durch eine Mehrzahl von Infrarotsensoren erfolgen, welche einen unterschiedlichen Erfassungsbereich abdecken. In anderen Ausführungsformen der Erfindung kann ein zweidimensionaler Infrarotsensor verwendet werden, welcher gegebenenfalls unter Zuhilfenahme einer abbildenden Optik, ein thermografisches Bild der im Erfassungsbereich befindlichen Personen bzw. der Personengruppe erzeugt. Auf diese Weise können die Oberflächentemperaturen unterschiedlicher Körperbereiche erfasst werden. Diese können Flächenbereiche der Körperperipherie und des Körperstammes umfassen und/oder bekleidete und unbekleidete Körperpartien. Aus den Temperatur-differenzen kann in einigen Ausführungsformen der Erfindung der Isolationswiderstand der Bekleidung bestimmt werden oder es kann aus der Bekleidungsart auf den Aktivitätslevel und/oder die thermische Vorgeschichte der zu erfassenden Personen geschlossen werden. Auch diese Maßnahmen erhöhen somit die Genauigkeit der Erfassung der thermischen Behaglichkeit.

In einigen Ausführungsformen der Erfindung kann eine Bilderfassungseinrichtung den Infrarotsensor enthalten. Dies kann in einigen Ausführungsformen der Erfindung dadurch realisiert werden, dass die Bilderfassungseinrichtung sowohl im sichtbaren als auch im infraroten Spektralbereich sensitiv ist und damit gleichzeitig Eingangsdaten für die Bildauswerteeinrichtung und aus der Infrarotstrahlung abgeleitete Temperaturdaten der Hautoberfläche der zu erfassenden Person an die Korrelationseinrichtung liefern kann.

In anderen Ausführungsformen der Erfindung kann die Bilderfassungseinrichtung ausschließlich im infraroten Spektralbereich, beispielsweise im Spektralbereich zwischen 5 µm und 50 µm oder zwischen 10 µm und 40 µm sensitiv sein. In diesem Fall steht ausschließlich ein Thermographiebild zur Verfügung, welches einerseits der Bildauswerteeinrichtung zugeführt wird, um eine Position im Raum und/oder die Gestik der Person zu erfassen und andrerseits die Temperaturen an verschiedenen Stellen des Körpergewebes und/oder die Körperkerntemperatur zu bestimmen. Diese Ausführungsform der Erfindung kann einfacher und/oder kostengünstiger aufgebaut und/oder betrieben werden.

In einigen Ausführungsformen der Erfindung enthält die Vorrichtung weiterhin eine Recheneinrichtung, mit welcher die Oberflächentemperatur zumindest eines Flächenelements bestimmbar ist, welches nicht im Erfassungsbereich der Bilderfassungseinrichtung und/oder des Infrarotsensors angeordnet ist. Diese Ausführungsform der Erfindung erlaubt die Erfassung aller die thermische Behaglichkeit beeinflussenden Faktoren, ohne dass der Raum flächendeckend mit Infrarotsensoren oder Bilderfassungseinrichtungen ausgestattet werden muss. Auf diese Weise kann die vorgeschlagene Vorrichtung mit geringerem Aufwand realisiert werden.

In einigen Ausführungsformen der Erfindung enthält die Vorrichtung weiterhin ein Klimatisierungssystem, welches die dem Raum zugeführte Luftmenge, die Lufttemperatur, die Luftfeuchte und/oder die Oberflächentemperatur zumindest einer Teilfläche der den Raum begrenzenden Flächen verändern kann. Das Klimatisierungssystem enthält weiterhin zumindest einen Regelkreis, mit welchem eine Oberflächentemperatur, eine Luftmenge, eine Luftfeuchte oder eine Lufttemperatur auf einen vorgebbaren Sollwert geregelt werden kann. Dieser Regelkreis kann die zumindest eine, die thermische Behaglichkeit repräsentierende Messgröße als Soll- oder Istwert zugeführt werden. Dies erlaubt die automatische Anpassung des Raumklimas an die jeweilige Aktivität oder die individuelle Konstitution oder Bekleidung des Benutzers. Auf diese Weise kann der Energieeinsatz zur Schaffung eines behaglichen Raumklimas reduziert werden, indem das Raumklima stets auf den individuellen Bedarf abgestimmt wird.

In einigen Ausführungsformen kann die vorgeschlagene Vorrichtung alternativ oder kumulativ auch zur visuellen Darstellung der thermischen Behaglichkeit, beispielsweise zu Forschungszwecken, und/oder zur Bestimmung der Körperkerntemperatur zumindest einer Person verwendet werden. In diesem Fall können erkrankte Personen in einem Personenkollektiv erkannt oder der Gesundheitszustand von Bewohnern eines Pflegeheimes fortwährend erfasst werden.

Nachfolgend soll die Erfindung anhand von Ausführungsbeispielen näher erläutert werden, welche in den anhängenden Figuren dargestellt sind. Dabei zeigt
- Figur 1: ein Blockschaltbild der erfindungsgemäßen Vorrichtung in einer ersten Ausführungsform.
- Figur 2: zeigt ein Blockschaltbild der erfindungsgemäßen Vorrichtung in einer zweiten Ausführungsform.
- Figur 3: zeigt einen mit der erfindungsgemäßen Vorrichtung ausgestatten Raum.

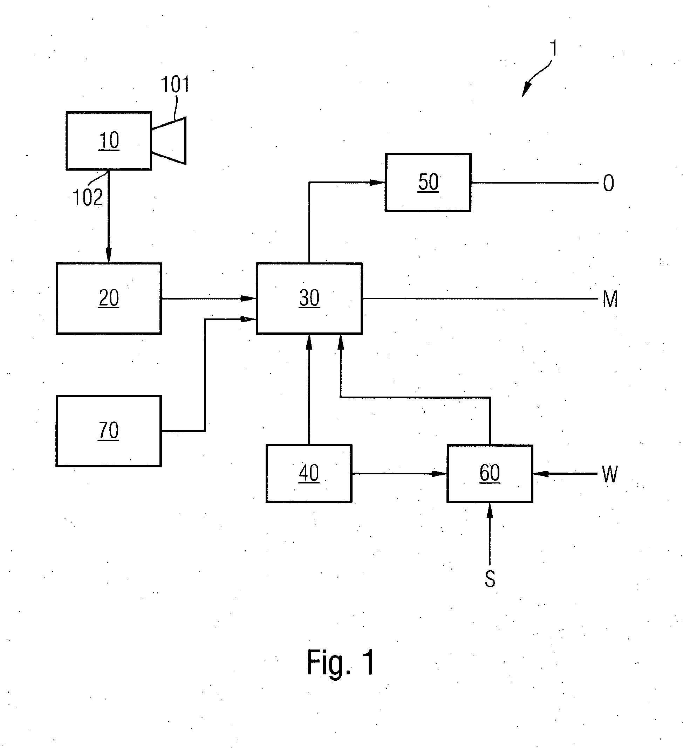

Figur 1 zeigt ein Blockschaltbild einer ersten Ausführungsform der vorliegenden Erfindung. Die Vorrichtung 1 enthält eine Bilderfassungseinrichtung 10. Die Bilderfassungseinrichtung 10 kann in einigen Ausführungsformen einen zweidimensionalen Bildsensor enthalten, beispielsweise einen CCD-Sensor. Der von der Bilderfassungseinrichtung 10 abgedeckte Raumbereich kann mittels einer abbildenden Optik 101 auf den Sensor abgebildet werden.

Die in Figur 1 dargestellte einzelne Bilderfassungseinrichtung 10 ist dabei nur beispielhaft zu verstehen. In anderen Ausführungsformen der Erfindung kann eine Mehrzahl von Bilderfassungseinrichtungen 10 zur stereoskopischen oder volumetrischen bzw. tomografischen Erfassung der Bewegung zumindest einer Person vorhanden sein. Die in Figur 1 dargestellte einzelne Bilderfassungseinrichtung 10 bezeichnet somit eine logische Einheit, nicht notwendigerweise die physikalisch vorhandene Anzahl an Geräten.

Die Bilderfassungseinrichtung 10 kann einen infraroten und optional einen sichtbaren Teil des elektromagnetischen Spektrums erfassen. Die Bilderfassungseinrichtung kann farbige oder schwarzweiße bzw. Graustufenbilder erzeugen. Die Bilderfassungseinrichtung 10 kann eine Mehrzahl von Bildern zyklisch oder ereignisgesteuert erzeugen.

In einigen Ausführungsformen der Erfindung kann die Bilderfassungseinrichtung einen A/D-Wandler enthalten, und so an ihrem Ausgang 102 eine Bilddaten repräsentierende, digitale Signalfolge bereitstellen.

Die Bilddaten der Bilderfassungseinrichtung 10 werden einer Bildauswerteeinrichtung 20 zugeführt. Die Bildauswerteeinrichtung 20 kann in einigen Ausführungsformen der Erfindung in Form einer Software ausgestaltet sein, welche auf einem Mikroprozessor oder einem Microcontroller ausgeführt wird. In anderen Ausführungsformen der Erfindung kann die Bildauswerteeinrichtung als integrierter Schaltkreis realisiert werden, beispielsweise als ASIC und/oder FPGA und/oder digitaler Signalprozessor.

Die Bildauswerteeinrichtung 20 ist dazu eingerichtet, aus dem Datenstrom der Bilderfassungseinrichtung zumindest eine Position im Raum und/oder eine Gestik und/oder eine Mimik und/oder anthropometrische Daten der zumindest einen Person zu erfassen. Sofern die Bilderfassungseinrichtung unterschiedliche Bilder zu unterschiedlichen Zeitpunkten erfasst, kann die Bildauswerteeinrichtung auch den zeitlichen Verlauf dieser Größen bestimmen. Die Bildauswerteeinrichtung kann hierzu eine Mustererkennung oder eine digitale Bildauswertung vornehmen.

Die von der Bildauswerteeinrichtung 20 generierten Daten repräsentieren somit ein Bewegungsprofil der zumindest einen Person im Raum, deren Gesten, deren Mimik, eine Körperhaltung und/oder weitere, hier nicht genannte verhaltensabhängige Personendaten. Diese werden nachfolgend der Korrelationseinrichtung 30 zugeführt.

Auch die Korrelationseinrichtung 30 kann in einigen Ausführungsformen der Erfindung als Software ausgeführt sein, weiche auf einem Mikroprozessor oder einem Microcontroller ausgeführt wird. In anderen Ausführungsformen der Erfindung kann die Korrelationseinrichtung 30 auch einen digitalen Signalprozessor, einen ASIC oder einem FPGA enthalten. Die Korrelationseinrichtung kann als neuronales Netz oder in Fuzzy-Logik realisiert werden.

Die Korrelationseinrichtung 30 hat die Aufgabe, aus den Daten der Bildauswerteeinrichtung zumindest eine, die thermische Behaglichkeit repräsentierende Messgröße zu erzeugen. Beispielsweise kann aus dem Bewegungsprofil des Benutzers im Raum eine Information über den Aktivitätsgrad der Person gewonnen werden. Bei einem hohen Aktivitätsgrad wird im Allgemeinen eine niedrigere Luftfeuchte und/oder eine niedrigere Lufttemperatur angenehmer empfunden. Bei einem niedrigeren Aktivitätsgrad können diese Größen angehoben werden, um die thermische Behaglichkeit zu erhöhen.

Aus der Mimik und/oder Gestik der erfassten Person kann ebenfalls auf die thermische Behaglichkeit geschlossen werden, etwa bei steigender Unruhe, Kältezittern oder wenn die Person Maßnahmen zur Änderung des Raumklimas ergreift, beispielsweise ein Fenster öffnet. Schließlich kann die Korrelationseinrichtung aus anthropometrischen Daten und/oder morphologischen Daten auf die thermische Behaglichkeit der jeweiligen Person schließen und/oder Modellparameter für ein thermophysiologisches Simulationsmodell liefern. In einigen Ausführungformen der Erfindung können die anthropometrischen Daten die Körpergröße zumindest einer Person enthalten. In einigen Ausführungsformen der Erfindung können die morphologischen Daten das Gewicht zumindest einer Person enthalten.

Weiterhin ist in Figur 1 ein Infrarotsensor 40 dargestellt, welcher die Temperatur der Haut- und/oder Bekleidungsoberfläche der Person erfasst. Durch die Verwendung eines Infrarotsensors kann die Temperaturmessung berührungslos erfolgen, sodass eine Verkabelung der im Raum befindlichen Personen mit Messfühlern nicht erforderlich ist. Auch die Temperaturdäten werden der Korrelationseinrichtung 30 zugeführt, sodass diese die thermische Behaglichkeit schneller und/oder mit größerer Genauigkeit feststellen kann. Auch der Infrarotsensor 40 kann mehrere Sensoren enthalten, welche unterschiedliche Raumbereiche erfassen oder identische Raumbereiche aus unterschiedlichen Richtungen erfassen, um eine dreidimensionale Temperaturerfassung zu ermöglichen. Sofern eine Mehrzahl von Temperatursensoren 40 vorhanden ist, können diese jeweils einen Punkt bzw. einen Raumwinkel erfassen oder eine räumlich aufgelöste Temperaturverteilung in ihrem Erfassungsbereich bestimmen.

Die Messwerte des Infrarotsensors 40 können in einigen Ausführungsformen einer optionalen Schätzeinrichtung 60 zugeführt werden, welche den Bekleidungsisolationswert der Personen im Raum bestimmt. Hierzu erhält die Schätzeinrichtung 60 Messwerte von zumindest einem bekleideten und einem unbekleideten Körperteil, sodass aus der Temperaturdifferenz und dem durch ein physiologisches Modell berechneten Wärmestrom auf den entsprechenden Bekleidungsisolationswert geschlossen werden kann. Die Genauigkeit des Bekleidungsisolationswertes kann erhöht werden, wenn der Schätzeinrichtung 60 optional auch Wetterdaten W und/oder Standortdaten S zugeführt werden. Hierdurch kann eine Vorauswahl des Bekleidungsisolationswertes getroffen werden und/oder der gemessene Wert plausibilisiert werden. So wird beispielsweise der Bekleidungsisolationswert im Durchschnitt bei warmer Witterung niedriger sein als bei kalter Witterung. Ebenso werden durchschnittliche Benutzer in einer Sporthalle andere Kleidung wählen als in einem Seminarraum oder einem Flughafen.

Auch die Schätzeinrichtung 60 kann durch eine Software realisiert sein. Die Software kann eine Fuzzy-Logik, ein Kennfeld oder ein neuronales Netz umfassen. Alternativ oder kumulativ kann die Schätzeinrichtung auch Hardwarekomponenten erhalten, wie beispielsweise einen A/D-Wandler, einen digitalen Signalprozessor, Halbleiterspeicher oder Mikroprozessor.

Die Genauigkeit des Bekleidungsisolationswertes kann genauer bestimmt werden, wenn in der Schätzeinrichtung ein thermophysiologisches Modell hinterlegt ist. Ein solches thermophysiologisches Modell kann die Thermoregulationseigenschaften wie Schwitzen, Kältezittern, vasomotorische Gefäßverengung und -erweiterung, den Stoffwechsel und/oder den Wärmehaushalt des menschlichen Körpers abbilden. Das Vorhandensein eines solchen Modells ist jedoch optional und kann in anderen Ausführungsformen der Erfindung auch entfallen.

Durch die Segmentierung ist bekannt, welche Körperteile infrarottechnisch gesehen werden, womit eine Differenzierung zwischen benachbarten bekleideten und unbekleideten Körperstellen vorgenommen werden kann und ferner beurteilt werden kann, zu welchen prozentualen Anteilen der Körper segmentweise bekleidet ist. Durch die Differenzierung und den parallelen Einsatz eines thermophysiologischen Systems kann bei bekanntem Wärmestrom nun auch der Bekleidungsisolationswert segmentweise berechnet werden.

Die Erfassung der Oberflächentemperaturen an bekleideten und unbekleideten Stellen einer Person mittels der Bilderfassungseinrichtung insbesondere unter Berücksichtigung der Körperposition erlaubt es, mit Hilfe eines mathematischen Modells Wärmeströme zu berechnen, die vom menschlichen Körper unter den aktuell vorherrschenden Umgebungsbedingungen an einem spezifischen Körperteil und/ oder am gesamten Köper abgegeben werden. Hierzu werden dem mathematischen Modell die aktuell gemessenen Umgebungsbedingungen (Luftfeuchtigkeit, lokale/ globale Oberflächentemperaturen, lokale/ globale Luftgeschwindigkeiten, Sitzflächentemperaturen usw.) als Inputparameter übergeben.

Das mathematische Modell selber ist dem menschlichen Thermoregulationssystem nachempfunden und bildet alle Thermoregulationsmechanismen (Schwitzen, Zittern, Gefäßverengung/ -erweiterung) mittels bekannter und validierter (Foda et al., 2011) Regressionsfunktionen ab.

Die Art der aktiven Thermoregulationsmechanismen (entweder Zittern, Schwitzen etc.) und deren Intensität sind wie beim realen Menschen stark von den vorherrschenden Umgebungsbedingungen abhängig. Als Folge dieser aktiven Regelmechanismen stellen sich beim mathematischen Modell entsprechende Hautoberflächentemperaturen ein, die dann mit den gemessenen Temperaturen an bekleideten und unbekleideten Oberflächensegmenten der Person verglichen werden können, da durch die Segmentierung bekannt ist zu welchem Anteil der Körper segmentweise bzw. global bekleidet ist (siehe hierzu auch Bekleidungsisolationswert Kapitel 1.1).

Befinden sich die Differenzen aus gemessenen und berechneten Temperaturen innerhalb eines gewissen Toleranzlevels, können Wärmeströme berechnet werden, die sich aus den entsprechenden Temperaturgradienten ergeben. Durch den Vergleich der berechneten Wärmeströme des mathematischen Modells und der aus der Messung der Oberflächentemperaturen berechneten Wärmeströme kann die Genauigkeit weiter verbessert werden.

Mit bekannten Temperaturen und Wärmeströmen kann eine Berechnung des Bekleidungswiderstands erfolgen, welcher dann in einen Bekleidungsisolationswert umgerechnet werden kann

Da der Bekleidungsisolationswert die thermische Behaglichkeit beeinflusst, kann die von der Korrelationseinrichtung 30 bereitgestellte Messgröße M eine verbesserte Genauigkeit aufweisen, wenn der Korrelationseinrichtung 30 der Ausgangswert der Schätzeinrichtung 60 zur Verfügung gestellt wird.

Schließlich kann in einigen Ausführungsformen der Erfindung eine Recheneinrichtung 50 vorgesehen sein, welcher Daten der Korrelationseinrichtung 30 zugeführt werden und welche dazu eingerichtet ist, die Oberflächentemperatur O zumindest eines Flächenelementes zu bestimmen, welches nicht im Erfassungsbereich der Bilderfassungseinrichtung und/oder des Infrarotsensors 40 angeordnet ist. Dadurch kann die Anzahl der Sensoren reduziert werden, ohne die Genauigkeit des Verfahrens zu beeinträchtigen.

Schließlich können der Korrelationseinrichtung 30 noch Messwerte des aktuellen Raumklimas zugeführt werden, welche mit Sensoren 70 erfasst werden, welche eine Lufttemperatur und/oder eine Luftfeuchte und/oder eine Strömungsgeschwindigkeit und/oder eine Luftmenge erfassen.

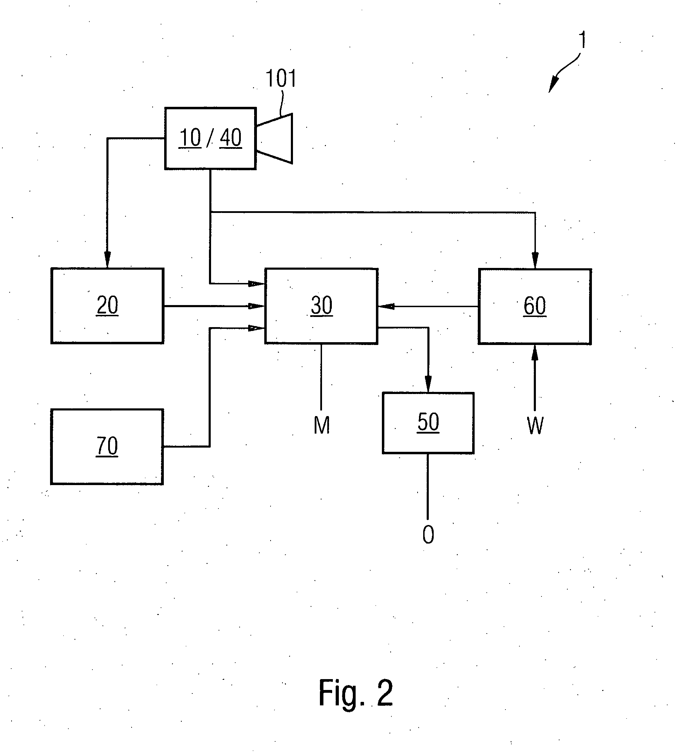

Figur 2 zeigt ein Blockschaltbild einer weiteren Ausführungsform der Erfindung. Gleiche Elemente sind mit gleichen Bezugszeichen versehen wie in Figur 1. Daher beschränkt sich die nachfolgende Beschreibung auf die Unterschiede der beiden Ausführungsformen.

Wie aus Figur 2 ersichtlich, enthält die Bilderfassungseinrichtung 10 einen Infrarotsensor 40. Dies kann in unterschiedlicher Weise realisiert werden. Beispielsweise kann der zu erfassende Raumbereich durch zumindest ein optisches Element 101 auf eine Ebene abgebildet werden. Der Strahlengang des optischen Elementes 101 kann einen Strahlteiler enthalten, sodass zumindest der infrarote Anteil des Spektrums auf den Infrarotsensor 40 trifft und zumindest der sichtbare Anteil des Spektrums auf die Bilderfassungseinrichtung 10.

In einer anderen Ausführungsform der Erfindung kann die Bilderfassungseinrichtung 10 und der Infrarotsensor 40 identisch sein, sodass auch die Bilderfassungseinrichtung die Erfassung der Bewegung zumindest einer Person durchführt. In diesem Fall kann die Erfassung der Bewegung ausschließlich durch die Erfassung des infraroten Anteils des elektromagnetischen Spektrums erfolgen. Auf diese Weise kann ein zusätzlicher Sensor zur Bilderfassung eingespart werden.

Da der von der Bilderfassungseinrichtung 10 und dem Infrarotsensor 40 erfasste Raumwinkel in beiden Fällen identisch ist, kann jedem Bildelement eine Temperatur zugeordnet werden. Hierdurch können bekleidete und unbekleidete Körperpartien mit größerer Genauigkeit anhand des Bewegungsmusters unterschieden werden. Gleichzeitig kann die Integration der Vorrichtung 1 in einem Raum aufgrund der geringeren Anzahl von Geräten unauffälliger erfolgen.

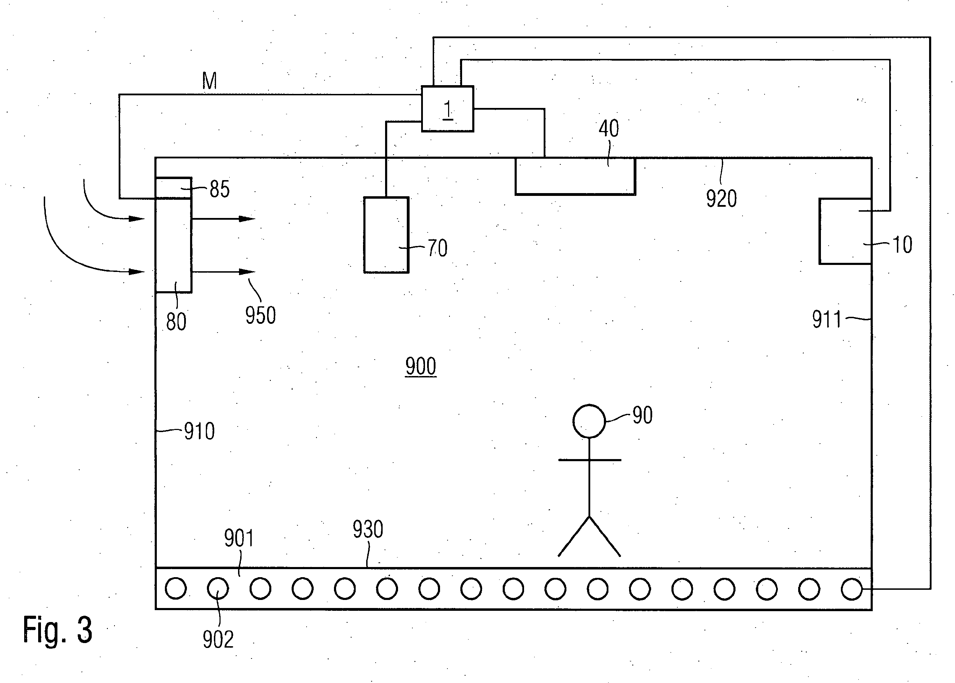

Figur 3 zeigt beispielhaft die Integration der Vorrichtung 1 in einem Raum 900. Bei dem in Figur 3 dargestellten Raum 900 handelt es sich um einen Innenraum in einem Gebäude. Der Raum 900 ist somit von einer Decke 920, Wänden 910 und einem Boden 930 begrenzt. Der Boden 930 ist mit einem Estrich 901 bedeckt, in welchen eine Heiz- und/oder Kühleinrichtung 902 integriert ist, um die Temperatur und damit die abgegebene Wärmestrahlung des Bodens 930 zu beeinflussen. In anderen Ausführungsformen der Erfindung kann eine ähnliche Heiz- und/oder Kühleinrichtung auch in einer Wand oder einer Decke angeordnet sein. In einer Ausführungsform der Erfindung kann die Heiz- und/oder Kühleinrichtung 902 Fluidkanäle enthalten, welche von einem Wärmeträgermedium höherer oder geringerer Temperatur durchflossen werden, um die Temperatur anzuheben oder abzusenken. In wiederum einer anderen Ausführungsform der Erfindung können die Fluidkanäle 902 bzw. die Heizeinrichtung auch entfallen. In ähnlicher Weise wie in Figur 3 für den Raum 900 eines Gebäudes dargestellt, kann die erfindungsgemäße Vorrichtung auch im Inneren eines Fahr- oder Flugzeuges oder eines Schiffes betrieben werden. In diesem Fall können auch personalisierte Heiz- Lüftungs- oder Kühleinrichtungen gesteuert oder geregelt werden, wie beispielsweise Sitzheizungen oder -belüftungen.

Weiterhin ist der Raum 900 mit einem optionalen Klimatisierungssystem 80 ausgestattet, welches einen Luftstrom 950 im Inneren des Raumes erzeugen kann. Über die Menge des Luftstromes 950 kann die Luftaustauschrate und die Strömungsgeschwindigkeit im Inneren des Raumes 900 beeinflusst werden. Über die Temperatur der eintretenden Luft 950 kann der Raum 900 beheizt oder gekühlt werden.

Die Ist-Werte der Lufttemperatur, der Strömungsgeschwindigkeit, der Luftfeuchte und/oder der Luftmenge werden durch Erfassungsmittel 70 erfasst. Die Messwerte werden der Vorrichtung 1 zugeführt, wie vorstehend anhand der Figuren 1 und 2 erläutert.

Weiterhin ist im Raum 900 zumindest eine Bilderfassungseinrichtung 10 angeordnet, mit welcher die Bewegung zumindest einer Person 90 erfasst werden kann. Auch die Daten der Bilderfassungseinrichtung 10 werden der Vorrichtung 1 zugeführt, wie bereits vorstehend erläutert. Schließlich befindet sich im Raum 900 zumindest ein Infrarotsensor 40, welcher zumindest ein Teilvolumen bzw. eine Teilfläche der Grundfläche des Raumes 900 abdeckt. Der Infrarotsensor 40 kann einerseits die Temperatur der Oberfläche der Person 90 an zumindest einer Stelle erfassen und diese Temperatur der Vorrichtung 1 zuführen. Andererseits kann der Infrarotsensor 40 auch die Temperatur des Bodens 930 und/oder der Wände 910 erfassen. Daneben können auch Flächenbereiche 911 vorhanden sein, deren Temperatur nicht unmittelbar von einem Infrarotsensor 40 gemessen wird.

Die Vorrichtung 1 kann nun Klimadaten bestimmen, bei welchen die Behaglichkeit der Person 90 maximal ist. Die Klimadaten, d.h. die Luftfeuchte, die Lufttemperatur oder auch die Strömungsgeschwindigkeit, bei welchen sich die maximale Behaglichkeit einstellt, können von der Anzahl der Personen 90, deren Geschlecht, deren Bekleidung, deren Alter oder der körperlichen Aktivität abhängig sein. In diesem Fall kann die Vorrichtung 1 in Abhängigkeit der genannten Größen unterschiedliche Sollwertvorgaben für das Raumklima erzeugen und Steuer-bzw. Regelsignale an die Steuer- bzw. Regeleinrichtung 85 ausgeben, welche das Klimatisierungssystem 80 und/oder die Heizeinrichtung 902 ansteuern, sodass sich das gewünschte Klima einstellt. Dadurch wird die subjektiv empfundene Behaglichkeit der zumindest einen Person 90 optimiert, ohne dass es einen manuellen Eingriffs in das Klimatisierungssystem 80 oder die Heiz- bzw. Kühleinrichtung 902 bedarf.

Selbstverständlich ist die Erfindung nicht auf die in den Figuren dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Ansprüche und die vorstehende Beschreibung "erste" und "zweite" Merkmale definieren, so dient diese Bezeichnung der Unterscheidung zweier gleichartiger Merkmale, ohne eine Rangfolge festzulegen.

## Patentansprüche

1. Vorrichtung (1) zur Erfassung der thermischen Behaglichkeit, enthaltend:
• einen Infrarotsensor (40), welcher dazu eingerichtet ist, ein thermographisches Bild zu erzeugen, indem die Temperatur an einer Mehrzahl von Stellen erfasst wird, zur Erfassung der Oberflächentemperatur zumindest einer Person (90),
• eine Bildauswerteeinrichtung (20), welcher die Daten des Infrarotsensors (40) zuführbar sind und welche dazu eingerichtet ist, die gemessenen Oberflächentemperaturen mit einem segmentierten physiologischen Modell der Person (90) zu korrelieren, wobei die Bildauswerteeinrichtung (20) zur Bestimmung einer Position im Raum und/oder einer Gestik und/oder anthropometrischer und/oder morphologischer Daten der zumindest einen Person (90) eingerichtet ist,
• eine Korrelationseinrichtung (30), welcher die Daten der Bildauswerteeinrichtung (20) zuführbar sind, und welche dazu eingerichtet ist, aus der Position in Raum und/oder der Gestik und/oder den anthropometrischen und/oder den morphologischen Daten der zumindest einen Person (90) zumindest eine die thermische Behaglichkeit repräsentierende Messgröße (M) zu erzeugen.

2. Vorrichtung nach Anspruch 1, weiterhin enthaltend zumindest eine Bilderfassungseinrichtung (10) zur Erfassung der Gestik und/oder der Mimik und/oder der Bewegung von zumindest einer Person (90) im Raum.

3. Vorrichtung nach Anspruch 1 oder 2, **dadurch gekennzeichnet, dass** die Bildauswerteeinrichtung und/oder der Infrarotsensor dazu eingerichtet ist, anthropometrische Daten und/oder Gestik und/oder Positionen einer Mehrzahl von Personen zu bestimmen und/oder
dass die Bildauswerteeinrichtung (20) dazu eingerichtet ist, den Aktivitätsgrad zumindest einer Person zu bestimmen und/oder
dass die Bildauswerteeinrichtung (20) dazu eingerichtet ist, eine morphologische und/oder anthropometrische Differenzierung der die thermische Behaglichkeit repräsentierende Messgröße (M) vorzunehmen und optional eine geschlechtsspezifische Bestimmung der die thermische Behaglichkeit repräsentierende Messgröße (M) vorzunehmen und/oder
dass die Bildauswerteeinrichtung (20) dazu eingerichtet ist, lokal unterschiedliche die thermische Behaglichkeit repräsentierende Messgrößen (M) zu bestimmen.

4. Vorrichtung nach einem der Ansprüche 1 bis 3, **dadurch gekennzeichnet, dass** die Bilderfassungseinrichtung und/oder der Infrarotsensor (40) dazu eingerichtet ist, Bilder zumindest einer Person fortlaufend zu erfassen, so dass mit der Bildauswerteeinrichtung der zeitliche Verlauf einer Position im Raum und/oder der zeitliche Verlauf der Gestik und/oder der zeitliche Verlauf der Mimik der zumindest einen Person bestimmbar ist.

5. Vorrichtung nach einem der Ansprüche 1 bis 4, **dadurch gekennzeichnet, dass** die Bilderfassungseinrichtung (10) den Infrarotsensor (40) enthält.

6. Vorrichtung nach einem der Ansprüche 1 bis 5, weiterhin enthaltend eine Recheneinrichtung (50), mit welcher die Oberflächentemperatur (O) zumindest eines Flächenelementes (911) bestimmbar ist, welches nicht im Erfassungsbereich der Bilderfassungseinrichtung (10) und/oder des Infrarotsensors (40) angeordnet ist.

7. Vorrichtung nach einem der Ansprüche 1 bis 6, weiterhin enthaltend ein Klimatisierungssystem (80), welches zumindest einen Regelkreis (85) enthält, welchem die zumindest eine die thermische Behaglichkeit repräsentierende Messgröße (M) als Soll- oder Istwert zuführbar ist.

8. Vorrichtung nach einem der Ansprüche 2 bis 7, weiterhin enthaltend eine Schätzeinrichtung (60), mittels welcher der Bekleidungsisolationswert der Person (90) bestimmbar ist, und welchem zumindest Wetterdaten (W) und/oder Standortdaten (S) und/oder die Daten des Infrarotsensors (40) zuführbar sind, wobei die Daten der Schätzeinrichtung (60) der Korrelationseinrichtung (30) zuführbar sind, wobei optional in der Schätzeinrichtung (60) ein thermophysiologisches Modell hinterlegt ist.

9. Vorrichtung nach einem der Ansprüche 1 bis 8, **dadurch gekennzeichnet, dass** die Bildauswerteeinrichtung (20) dazu eingerichtet ist, die thermische Vorgeschichte der Person (90) anhand von Bekleidungsgrad und/ oder Außentemperatur und/ oder geschlechtsspezifischen Abschätzungen und/oder alterspezifischen Merkmalen und/oder anthropometrischen Merkmalen und/ oder morphologischen Merkmalen und/oder durch die parallele Anwendung eines thermophysiologischen Modells zu ermitteln.

10. Verfahren zur Erfassung der thermischen Behaglichkeit, enthaltend die folgenden Schritte:
• Erzeugen eines thermographischen Bildes zur Erfassung der Oberflächentemperatur zumindest einer Person (90), indem die Temperatur an einer Mehrzahl von Stellen mit einem Infrarotsensor (40) erfasst wird, ,
• zuführen der Daten des Infrarotsensors (40) zu einer Bildauswerteeinrichtung (20), Erzeugen eines segmentierten Oberflächenmodells der Person (90) aus der Oberflächentemperatur und Bestimmung einer Position im Raum und/oder einer Gestik und/oder anthropometrischer und/oder morphologischer Daten der zumindest einen Person (90),
• zuführen der Daten der Bildauswerteeinrichtung (20) zu einer Korrelationseinrichtung (30), mit welcher aus der Position in Raum und/oder der Gestik und/oder der anthropometrischen Daten und/oder der morphologischen Daten der zumindest einen Person (90) zumindest eine die thermische Behaglichkeit repräsentierende Messgröße (M) erzeugt wird.

11. Verfahren nach Anspruch 10, weiterhin enthaltend den folgenden Schritt: Erfassen der Gestik und/oder der Mimik und/der der Bewegung von zumindest einer Person (90) im Raum mittels zumindest einer Bilderfassungseinrichtung (40) und Zuführen der Daten der Bilderfassungseinrichtung zu der Korrelationseinrichtung (30).

12. Verfahren nach einem der Ansprüche 10 oder 11, weiterhin enthaltend die folgenden Schritte: Bestimmen eines Bekleidungsisolationswertes der Person (90) mit einer Schätzeinrichtung (60), welcher zumindest Wetterdaten (W) und/oder Standortdaten (S) und/oder die Daten des Infrarotsensors (40) zugeführt werden und Zuführen der Daten der Schätzeinrichtung (60) zu der Korrelationseinrichtung (30).

13. Verfahren nach einem der Ansprüche 10 bis 12, **dadurch gekennzeichnet, dass** der Aktivitätsgrad zumindest einer Person bestimmt wird und/oder
dass zumindest eine Temperaturdifferenz zwischen einem bekleideten und einem unbekleideten Körperteil zumindest einer Person bestimmt wird und aus den Temperaturdifferenzen und dem mit Hilfe eines physiologischen Modells berechneten, äquivalenten Wärmestrom der Isolationswiderstand der Bekleidung bestimmt wird.

14. Verfahren nach einem der Ansprüche 10 bis 13, **dadurch gekennzeichnet, dass** zumindest eine Temperaturdifferenz zwischen einem bekleideten und einem unbekleideten Körperteil zumindest einer Person bestimmt wird und aus der Bekleidungsart auf den Aktivitätslevel und/oder die thermische Vorgeschichte der zumindest einen Person geschlossen wird.

15. Verwendung einer Vorrichtung nach einem der Ansprüche 1 bis 9 zur visuellen Darstellung der thermischen Behaglichkeit und/oder zur Bestimmung einer Körpertemperatur der Person (90) und/oder zur Steuerung oder Regelung des Raumklimas.

## Claims

1. Apparatus (1) for detecting thermal comfort, containing:
• an infrared sensor (40), which is adapted to produce a thermographic image by collecting the temperature at a plurality of spots, for detecting the surface temperature of at least one person (90),
• an image evaluation device (20), which can be supplied with the data of the infrared sensor (40) and which his adapted to correlate the measured surface temperatures with a segmented physiological model of the person (90), wherein the image evaluation device (20) is adapted to determine a position in the room and/or gestures and/or the anthropometric and/or the morphological data of the at least one person (90),
• a correlation device (30), which can be supplied with the data of the image evaluation device (20) and which is adapted to produce at least one measured variable (M) which represents thermal comfort from the position in the room and/or the gestures and/or the anthropometric and/or morphological data of the at least one person (90).

2. Apparatus according to claim 1, further containing at least one image capturing device (10) for collecting the gestures and/or the facial expressions and/or the movement of at least one person (90) in the room.

3. Apparatus according to claim 1 or 2, **characterized in that** the image evaluation device and/or the infrared sensor is adapted to determine anthropometric data and/or gestures and/or positions of a plurality of persons and/or
that the image evaluation device (20) is adapted to determine the activity level of at least one person and/or
that the image evaluation device (20) is adapted to make a morphological and/or anthropometric differentiation of the measured variable (M) representing thermal comfort and optionally masking a gender-related determination of the measured variable (M) representing thermal comfort and/or
that the image evaluation device (20) is adapted to determine locally different measured variables (M) representing thermal comfort.

4. Apparatus according to any of claims 1 to 3, **characterized in that** the image capturing device and/or the infrared sensor (40) is adapted to continuously collect images of at least one person so as to determine the time course of a position in the room and/or the time course of the gestures and/or the time course of the facial expressions of the at least one person by means of the image evaluation device.

5. Apparatus according to any of claims 1 to 4, **characterized in that** the image capturing device (10) contains the infrared sensor (40).

6. Apparatus according to any of claims 1 to 5, further containing a computing device (50), by means of which the surface temperature (O) of at least one surface element (911) can be determined which is not arranged in the capturing area of the image capturing device (10) and/or of the infrared sensor (40).

7. Apparatus according to any of claims 1 to 6, further containing an air-conditioning system (80) which contains at least one control circuit (85) which can be supplied with the at least one measured variable (M) representing thermal comfort as a desired or actual value.

8. Apparatus according to any of claims 2 to 7, further containing an estimation device (60), by means of which the clothing insulation value of the person (90) can be determined and which can be supplied with at least weather data (W) and/or location data (5) and/or the data of the infrared sensor (40), wherein the data of the estimation device (60) can be supplied to the correlation device (30), wherein a thermophysiological model is optionally deposited in the estimation device (60).

9. Apparatus according to any of claims 1 to 8, **characterized in that** the image evaluation device (20) is adapted to determine the thermal background of the person (90) by means of clothing extent and/or outside temperature and/or gender-related estimations and/or agerelated features and/or anthropometric features and/or morphological features and/or by the parallel use of a thermophysiological model.

10. Method for detecting thermal comfort, containing the steps of:
• producing a thermographic image for detecting the surface temperature of at least one person (90) by collecting the temperature at a plurality of spots by means of an infrared sensor (40),
• supplying the data of the infrared sensor (40) to an image evaluation device (20), producing a segmented surface model of the person (90) from the surface temperature and determining a position in the room and/or gestures and/or anthropometric and/or morphological data of the at least one person (90),
• supplying the data of the image evaluation device (20) to a correlation device (30), by means of which at least one measured variable (M) representing thermal comfort is produced from the position in the room and/or the gestures and/or the anthropometric data and/or the morphological data of the at least one person (90).

11. Method according to claim 10, further containing the step of:
collecting the gestures and/or the facial expressions and/or the movement of at least one person (90) in the room by means of at least one image capturing device (40) and supplying the data of the image capturing device to the correlation device (30).

12. Method according to any of claims 10 or 11, further containing the steps of: determining a clothing insulation value of the person (90) by means of an estimation device (60) which is supplied with at least weather data (W) and/or location data (S) and/or the data of the infrared sensor (40) and supplying the data of the estimation device (60) to the correlation device (30).

13. Method according to any of claims 10 to 12, **characterized by** determining the activity level of at least one person and/or
by determining at least one temperature difference between a clothed part of the body and an unclothed one of at least one person and by determining the insulation resistance of the clothing from the temperature differences and the equivalent heat flow calculated by means of a physiological model.

14. Method according to any of claims 10 to 13, **characterized by** determining at least one temperature difference between a clothed part of the body and an unclothed one of at least one person and concluding the activity level and/or the thermal background of the at least one person from the type of clothing.

15. Use of an apparatus according to any of claims 1 to 9 for the visual representation of thermal comfort and/or for determining a body temperature of the person (90) and/or for controlling or regulating the indoor climate.

## Revendications

1. Dispositif (1) pour la détermination du confort thermique, comprenant :
- un capteur à infrarouge (40), qui est conçu pour produire une image thermographique en détectant la température à une pluralité de points, afin de détecter la température de surface d'au moins une personne (90),
- un système d'exploitation d'image (20), auquel les données du capteur infrarouge (40) peuvent être amenées, et qui est conçu pour mettre en corrélation les températures de surface mesurées avec un modèle physiologique segmenté de la personne (90), dans lequel le système d'exploitation d'image (20) est conçu pour déterminer une position dans l'espace et/ou un geste et/ou des données anthropométriques et/ou morphologiques de ladite au moins une personne (90),
- un système de corrélation (30), auquel les données du système d'exploitation d'image (20) peuvent être amenées, et qui est conçu pour engendrer, à partir de la position dans l'espace et/ou du geste et/ou des données anthropométriques et/ou des données morphologiques de ladite au moins une personne (90), au moins une grandeur de mesure (M) représentant le confort thermique.

2. Dispositif selon la revendication 1, contenant en outre au moins un système de détection d'image (10) pour détecter le geste et/ou la mimique et/ou le mouvement d'au moins une personne (90) dans l'espace.

3. Dispositif selon la revendication 1 ou 2, **caractérisé en ce que** le système d'évaluation d'image et/ou le capteur à infrarouge est conçu pour déterminer des données anthropométriques et/ou des gestes et/ou des positions d'une pluralité de personnes, et/ou
**en ce que** le système d'évaluation d'image (20) est conçu pour déterminer le degré d'activité d'au moins une personne, et/ou en ce que le système d'évaluation d'image (20) est conçu pour procéder à une différentiation morphologique et/ou anthropométrique de la grandeur de mesure (M) représentant le confort thermique, et pour procéder à une détermination spécifique vis-à-vis du sexe sur la grandeur de mesure (M) représentant le confort thermique, et/ou en ce que le système d'évaluation d'image (20) est conçu pour déterminer localement différentes grandeurs de mesure (M) représentant le confort thermique.

4. Dispositif selon l'une des revendications 1 à 3, **caractérisé en ce que** le système de détection d'image et/ou le capteur à infrarouge (40) est conçu pour détecter des images d'au moins une personne en continu, de sorte qu'il est possible de déterminer au moyen du système d'évaluation d'image l'évolution temporelle d'une position dans l'espace et/ou l'évolution temporelle d'un geste et/ou l'évolution temporelle de la mimique de ladite au moins une personne.

5. Dispositif selon l'une des revendications 1 à 4, **caractérisé en ce que** le système de détection d'image (10) contient le capteur à infrarouge (40).

6. Dispositif selon l'une des revendications 1 à 5, comprenant en outre un système de calcul (50) au moyen duquel il est possible de déterminer la température de surface (O) d'au moins un élément surfacique (911) qui n'est pas agencé dans la zone de détection du système de détection d'image (10) et/ou du capteur à infrarouge (40).

7. Dispositif selon l'une des revendications 1 à 6, comprenant en outre un système de climatisation (80), qui inclut au moins un circuit de régulation (85) auquel peut être amenée ladite au moins une grandeur de mesure (M) représentant le confort thermique, à titre de valeur de consigne ou de valeur réelle.

8. Dispositif selon l'une des revendications 2 a 7, comprenant en outre un moyen d'estimation (60), au moyen duquel la valeur d'isolation vestimentaire de la personne (90) peut être déterminée, et auquel peuvent être amenées au moins des données météorologiques (W) et/ou des données locales (S) et/ou les données du capteur à infrarouge (40), les données du moyen d'estimation (60) pouvant être amenées au système de corrélation (30), et un modèle thermophysiologique est mémorisé en option dans le moyen d'estimation (60).

9. Dispositif selon l'une des revendications 1 à 8, **caractérisé en ce que** le système d'évaluation d'image (20) est conçu pour déterminer l'historique antérieur thermique de la personne (90) au moyen de l'importance de son habillage et/ou de la température extérieure et/ou d'estimations spécifiques à son sexe et/ou des caractéristiques spécifiques à son âge et/ou des caractéristiques anthropométriques et/ou des caractéristiques morphologiques et/ou par application parallèle d'un modèle thermophysiologique.

10. Procédé pour détecter le confort thermique, comprenant les étapes suivantes consistant à :
- engendrer une image thermographique pour détecter la température de surface d'au moins une personne (90), en détectant la température à une pluralité de points au moyen d'un capteur infrarouge (40),
- amener les données du capteur à infrarouge (40) à un système d'évaluation d'image (20), engendrer un modèle de surface segmenté de la personne (90) à partir de la température de surface et de la détermination d'une position dans l'espace et/ou d'un geste et/ou de données anthropométriques et/ou morphologiques de ladite au moins une personne (90),
- amener les données du système d'évaluation d'image (20) à un système de corrélation (30) au moyen duquel on engendre, à partir de la position dans l'espace et/ou du geste et/ou des données anthropométriques et/ou des données morphologiques de ladite au moins une personne (90), au moins une grandeur de mesure (M) représentant le confort thermique.

11. Procédé selon la revendication 10, comprenant en outre les étapes suivantes consistant à détecter le geste et/ou la mimique et/ou le mouvement d'au moins une personne (90) dans l'espace au moyen d'au moins un système de détection d'image (40), et à amener les données du système de détection d'image au système de corrélation (30).

12. Procédé selon l'une des revendications 10 ou 11, comprenant en outre les étapes suivantes consistant à déterminer une valeur d'isolation vestimentaire de la personne (90) avec un moyen d'estimation (60), auquel on amène au moins des données météorologiques (W) et/ou des données locales (S) et/ou les données du capteur à infrarouge (40), et à amener les données du moyen d'estimation (60) au système de corrélation (30).

13. Procédé selon l'une des revendications 10 à 12, **caractérisé en ce que** l'on détermine le degré d'activité d'au moins une personne et/ou en ce que l'on détermine au moins une différence de température entre une partie corporelle habillée et une partie corporelle non-habillée d'au moins une personne, et **en ce que** l'on détermine, à partir des différences de température et du flux thermique équivalent calculé avec l'aide d'un modèle physiologique, la résistance d'isolation des vêtements.

14. Procédé selon l'une des revendications 10 à 13, **caractérisé en ce que** l'on détermine au moins une différence de température entre une partie corporelle habillée et une partie corporelle non-habillée d'au moins une personne et, **en ce que** l'on conclut quant au niveau d'activité et/ou quant à l'historique thermique antérieur de ladite au moins une personne à partir du type de vêtements.

15. Utilisation d'un dispositif selon l'une des revendications 1 à 9 pour la représentation visuelle du confort thermique et/ou pour la détermination d'une température corporelle de la personne (90) et/ou pour la commande ou la régulation des conditions climatiques locales.
